## Europäisches Patentamt
## European Patent Office
## Office européen des brevets

(19)

(11) Veröffentlichungsnummer: **0 237 053 B1**

(12)

# EUROPÄISCHE PATENTSCHRIFT

(45) Veröffentlichungstag der Patentschrift:
19.06.91 Patentblatt 91/25

(51) Int. Cl.⁵: **A61F 2/34**, A61L 27/00

(21) Anmeldenummer: 87103530.9

(22) Anmeldetag: 11.03.87

(54) Einteilige zementfrei verankerbare biokompatible Hüftgelenkpfanne.

(30) Priorität: 12.03.86 DD 287801

(43) Veröffentlichungstag der Anmeldung:
16.09.87 Patentblatt 87/38

(45) Bekanntmachung des Hinweises auf die
Patenterteilung:
19.06.91 Patentblatt 91/25

(84) Benannte Vertragsstaaten:
AT BE CH DE FR GB IT LI NL SE

(56) Entgegenhaltungen:
EP-A- 0 211 676
DE-A- 3 446 048
FR-A- 2 310 322
FR-A- 2 548 661

(73) Patentinhaber: Technische Universität
Chemnitz
Postfach 964
O-9010 Chemnitz (DE)

(72) Erfinder: Kurze, Peter, Dr.sc.nat.
Hauptstrasse 48
O-9109 Oberlichtenau (DE)
Erfinder: Rabending, Klaus, Dr.rer.nat.
Arthur-Beil-Strasse 44
O-9115 Taura (DE)
Erfinder: Krysmann, Waldemar, Dr. rer.nat.
Fritz-Schmenkel-Strasse 39
O-9071 Karl-Marx-Stadt (DE)
Erfinder: Daniel, Peter, Dr. Sc.med.
Leipziger Strasse 175
O-9081 Karl-Marx-Stadt (DE)
Erfinder: Wehner, Wilfried, OMR
Prof.Dr.sc.Med.
Max-Planck-Strasse 46b
O-9081 Karl-Marx-Stadt (DE)
Erfinder: Polster, Manfred
Kirchhoffstrasse 27
O-9030 Karl-Marx-Stadt (DE)
Erfinder: Morgenstern, Rainer, Dr.med.
Enzmannstrasse 15
O-9005 Karl-Marx-Stadt (DE)

(74) Vertreter: Spott, Gottfried, Dr. et al
Sendlinger-Tor-Platz 11
W-8000 München 2 (DE)

## Beschreibung

Die Erfindung betrifft eine einteilige zementfrei verankerbare biokompatible Hüftgelenkpfanne für die Verwendung als Endoprothese.

Die in der Chirurgie verwendeten Hüftgelenkpfannen bestehen aus Plaste, vorzugsweise polyethylen, oder Metall mit polyethyleneinsatz oder Vollkeramik.

Das für Hüftgelenkpfannen eingesetzte polyethylen (DE-OS 3200340, CH-P 64747) ist in seifer kompaktheit relativ gut biokompatibel. Es zeigt sich aber, daß der Abrieb, verursacht durch die Einwirkung des Gelenkkopfes, vom Körper schwer oder nicht resorbiert wird. Dadurch können Entzündungen hervorgerufen werden, die Reoperationen nach sich ziehen. Trotz des aufgebrachten Verankerungsprofils an der Gelenkpfanne aus polyethylen (CH-P 648747) gelingt nur eine unzureichende Langzeitstabilisierung.

Günstiger lassen sich Zweikomponentenhüftgelenkpfannen, bestehend aus Metall und Polyethyleneinsatz (DE-OS 3214773) mit dem Knochen formschlüssig verankern. Allerdings bleibt der Nachteil des Einsatzes von Polyethylen, wie vorher beschrieben, bestehen.

In AT-P 337885 wird ein implantierbarer künstlicher Gelenkersatz aus Keramik vorgeschlagen, wobei Kopf und Pfanne aus Keramik bestehen. Trotz der guten Biokompatibilität der Keramik, der Resorbierbarkeit des Abriebes und der günstigen Gleitpaarungen, bleiben wie bei allen auch vorher beschriebenen Systemen, die geometrisch bedingten Nachteile bestehen. Das sind insbesondere die großen Abmessungen der Gelenkpfannen im Verhältnis zur Beckenanatomie.

Oftmals sind dadurch zusätzliche Operationen, wie Beckenosteotomien nach Chiari oder Pfannendachplastiken nach Harris, zur sicheren Verankerung notwendig. Dysplasiehüften können mit den bisher angebotenen Lösungen nicht versorgt werden.

Der Erfindung liegt die Aufgabe zugrunde, eine universell einsetzbare einteilige, zementfrei sicher verankerbare Hüftgelenkpfanne mit hoher Biokompabilität, d.h. eine möglichst kleinvolumige, der Anatomie des menschlichen Beckens angepaßte, mit guten Gleiteigenschaften versehene, mechanisch stabile Hüftgelenkpfanne zu schaffen. Eine diese Eigenschaften aufweisende Hüftgelenkpfanne zeichnet sich durch eine lange Liegedauer aus und kann mit bekannter Operationstechnik eingesetzt werden, wobei Reoperationen wegen Pfannenlockerung oder infolge von Entzündungen reduziert werden können.

Erfindungsgemäß wird die Aufgabe dadurch gelöst, daß die einteilige Gelenkpfanne aus Tantal, Titanium oder Legierungen hiervon geformt ist, die einen ihren Umfang mindestens zu 2/3 umfassenden perforierten Bördelring für die Verankerung aufweist und auf der dem Beckenknochen zugewandten Oberfläche eine arteigene Oxidschicht mit Poren einem Kalziumphosphatgehält > 40% besitzt sowie auf der gelenkseitigen Oberfläche der Pfanne einen Zweischichtverbund trägt, der metallseitig eine mechanisch verdichtete feinstporige, arteigene Oxidschicht und darauf eine feinporige, arbriebfeste, an Kalziumphosphat 10-30% enthaltene, arteigene, oxidische Gleitschicht mit Poren enthält.

Metalle wie Titanium und Tantal bilden Oxidschichten im Sinne von Sperrschichten aus.

Die Vorteile der erfindungsgemäßen Lösung bestehen darin, daß eine Minimierung der Gelenkpfannenmaße bei Garantie einer hohen mechanischen Stabilität gegeben ist. Dadurch ist auch eine sichere Verankerung im displastischen Hüftgelenkbereich möglich. Die Implantation erfordert kein zusätzliches Implantationsinstrumentarium, erfordert keinen Knochenzement sowie keine zusätzliche Operation wie Beckenosteotomien oder Pfannendachplastiken. Der spezielle Schichtaufbau garantiert eine hohe Biokompatilität. Die Schicht auf der dem Beckenknochen zugewandten Seite ist knochenwachstumsfördernd und in ihrer geometrischen Struktur so beschaffen, daß sich das wachsende Gewebe in den Flaschenporen knopfdruckartig verankert.

Der auf der gelenkseitigen Oberfläche der Pfanne befindliche Zweischichtverbund minimiert einerseits die Diffusion von Metallionen und den Abrieb und schafft andererseits günstige Voraussetzungen für Speicherung und Transport von Gelenkflüssigkeit. Außerdem sind schmierwirksame Oberflächenfilme aus Phosphaten oder Oxiden vorhanden, deren Abrieb nachgewiesenermaßen vom Organismus problemlos resorbiert wird.

Die erfindungsgemäße Lösung ermöglicht eine Langliegedauer in menschlichen Körper und verhindert implantatbedingte Reoperationen. Dadurch ist es möglich, jüngere Patienten mit einer Hüftgelenkendoprothese zu versorgen.

Die Erfindung soll nachstehend an einem Ausführungsbeispiel näher erläutert werden.

Eine aus Reinsttitanblech hergestellte Hüftgelenkfanne mit einem Innenradius von 16 mm und einer Gesamtwandstärke von 1,08 mm besitzt einen 8 mm breiten, mindestens zu 2/3 die Zirkonferenz umfassenden Bördelring, in dem sich vier gleichmäßig verteilte Bohrungen von 4, 5 mm befinden.

Die dem Beckenknochen zugewandte Oberfläche trägt eine 20 μm starke ANOF-Oxidschicht (ANOF = Verfahren der anodischen Oxidation unter Funkenentladung) mit einem Kalziumphisphatgehalt an der Randzone von 45% und einer relativen Oberfläche von 540%. 25% der Poren besitzen eine Flaschengestalt.

Die gelenkseitige Oberfläche der Pfanne hat zum Metall hin eine 2 μm starke mechanisch verdichtete Oxidschicht. Darauf befindet sich eine 8 μm starke ANOF-Oxidschicht mit 15% Kalziumphosphat in der Randzone. Die relative Oberfläche ist > 550%. Die Porengestalt entspricht vorwiegend der Trichterform.

8 Wochen nach der Operation mit dieser Gelenkpfanne zusammen mit einer keramikkugelkopftragenden Endoprothese war die Einheilung des Implantates abgeschlossen und die Belastung ohne Stützstöcks freigegeben.

## Ansprüche

1. Einteilige, zementfrei verankerbare biokompatible Hüftgelenkpfanne für die Kombination mit einem Hüftgelenkkopf, dadurch gekennzeichnet, daß die Gelenkpfanne aus Tantal, Titanium oder Legierungen hiervon geformt ist, die einen ihren Umfang mindestens zu 2/3 umfassenden perforierten Bördelring für die Verankerung aufweist, und auf der dem Beckenknochen zugewandten Oberfläche eine arteigene Oxidschicht mit Poren und einem Kalziumphosphatgehalt größer 40% besitzt sowie auf der gelenkseitigen Oberfläche der Pfanne einen Zweischichtverbund trägt, der metallseitig eine mechanisch verdichtete feinstporige arteigene Oxidschicht und darauf eine feinporige abriebfeste 10-30% Kalziumphosphat enthaltende arteigene oxidische Gleitschicht mit Poren enthält.

2. Einteilige, zementfrei verankerbare, biokompatible Hüftgelenkpfanne nach Anspruch 1, dadurch gekennzeichnet, daß die Gelenkpfanne aus Blech der Metalle Tantal, Titanium oder Legierungen hiervon besteht.

3. Einteilige, zementfrei verankerbare, biokompatible Hüftgelenkpfanne nach Anspruch 1, dadurch gekennzeichnet, daß die Gelenkpfanne ein Sinterkörper aus Tantal, Titanium oder Legierungen hiervon ist.

4. Einteilige, zementfrei verankerbare, biokompatible Hüftgelenkpfanne nach Anspruch 1 bis 3, dadurch gekennzeichnet, daß die Poren Flaschen- und/oder Zylinder- und/oder Trichterporen sind.

## Claims

1. A one-piece non-cementitious anchorable and bio-compatible acetabular cup to be combined with an acetabular condyle, characterized in that the acetabular cup is made from tantalum, titanium or alloys thereof and comprises along at least two thirds of its circumference a perforated flanged ring for the anchorage and is provided on its surface facing the pelvic bone with a characteristic oxide layer having pores and containing more than 40% of calcium phosphate and carries on the cup surface proximal to the joint a duplex laminate, the metallic side of said duplex laminate carrying a mechanically consolidated superfine-pored characteristic oxide layer and thereupon a fine-pored non-abrasive characteristic oxide sliding layer having pores and containing 10 to 30% of calcium phosphate.

2. The one-piece non-cementitious anchorable and bio-compatible acetabular cup of claim 1, characterized in that the acetabular cup is a sheet metal consisting of tantalum, titanium or alloys thereof.

3. The one-piece non-cementitious anchorable and bio-compatible acetabular cup of claim 1, characterized in that the acetabular cup is a sintered body made from tantalum, titanium or alloys thereof.

4. The one-piece non-cementitious anchorable and bio-compatible acetabular cup of claims 1 to 3, characterized in that the pores are bottle-shaped pores and/or cylindrical pores and/or funnel-shaped pores.

## Revendications

1. Cupule cotyloïdienne biocompatible, monobloc, ancrable sans ciment, à associer à une tête fémorale, caractérisée en ce que la cupule cotyloïdienne est en tantale, titane ou alliages de ces matériaux, qu'elle présente pour son ancrage une collerette perforée englobant au moins les 2/3 de sa circonférence, qu'elle possède sur sa surface tournée vers l'os pelvien une couche d'oxyde particulière dotée de pores et ayant une teneur en phosphate de calcium supérieure à 40%, et qu'elle porte sur sa surface côté articulation un ensemble de deux couches qui comprend côté métal une couche d'oxyde particulière, à pores très fins, comprimée mécaniquement, surmontée d'une couche lubrifiante oxydée, particulière, à pores fins, résistant à l'abrasion et contenant 10 à 30% de phosphate de calcium.

2. Cupule cotyloïdienne biocompatible, monobloc, ancrable sans ciment selon la revendication 1, caractérisée en ce que la cupule cotyloïdienne est en tôle des métaux tantale, titane ou en alliages de ces métaux.

3. Cupule cotyloïdienne biocompatible, monobloc, ancrable sans ciment selon la revendication 1, caractérisée en ce que la cupule cotyloïdienne est un corps fritté en tantale, titane ou en alliages de ces métaux.

4. Cupule cotyloïdienne biocompatible, monobloc, ancrable sans ciment selon les revendications 1 à 3, caractérisée en ce que les pores ont la forme d'une bouteille et/ou d'un cylindre et/ou d'un entonnoir.